# EUROPÄISCHE PATENTSCHRIFT

(11) **EP 0 667 164 B1**
(45) Veröffentlichungstag und Bekanntmachung des Hinweises auf die Patenterteilung: **29.12.1999**
(21) Anmeldenummer: 94102079.4
(22) Anmeldetag: 10.02.1994
(51) Int. Cl.: A61K 38/46

(54) **Verwendung hydrolytischer Enzyme zur Verringerung bzw. Vermeidung der Nebenwirkungen von Bleomycin**
Use of hydrolytic enzyme for reducing or preventing side effects of bleomycine
Utilisation d'enzyme hydrolytique pour réduire ou empêcher les effets secondaires de la bléomycine

(43) Veröffentlichungstag der Anmeldung: 16.08.1995
(73) Patentinhaber: MUCOS EMULSIONSGESELLSCHAFT m.b.H., D-13509 Berlin (DE)
(72) Erfinder: Ransberger, Karl, Dr.,, D-82042 Seeshaupt, (DE); Schedler, Michael W., Dr.,, D-66482 Zweibrücken, (DE); Stauder, Gerhard, Dr.,, D-82515 Wolfrathausen, (DE)
(74) Vertreter: Grünecker, Kinkeldey, Stockmair & Schwanhäusser Anwaltssozietät

(56) Entgegenhaltungen:
- WO-A-92/02230
- DE-A- 1 951 822
- CHEST, Bd.82, Nr.2, August 1982 Seite 219 N. HIROSE ET AL. 'CLINICAL AND EXPERIMENTAL STUDY ON THE PREVENTION OF BLEOMYCIN-INDUCED LUNG INJURY BY UROKINASE'

## Beschreibung

Die vorliegende Erfindung betrifft die Verwendung von mindestens einem hydrolytischen Enzym zur Herstellung eines Arzneimittels zur Verringerung bzw. Vermeidung der durch eine Behandlung mit Bleomycin hervorgerufenen Thrombocytopenie.

Die Dosierung von Bleomycin im Rahmen einer chemotherapeutischen Behandlung ist häufig limitiert durch die von Bleomycin ab einer bestimmten Konzentration hervorgerufenen Nebenwirkungen. Zu den Nebenwirkungen von Bleomycin zählt dessen Pulmotoxizität, die in 3 bis 40 % der Fälle, bei denen Bleomycin verwendet wird, beobachtet wird (Balikian, J. P. et al. 1982, AJR 139, 455-461). Zu den Risikofaktoren für das Auftreten von Lungenveränderungen zählt eine Bleomycin-Gesamtdosis von mehr als 250 mg. Jedoch müssen auch weit niedrigere Bleomycin-Dosen als potentiell pulmotoxisch betrachtet werden.

Als weitere Nebenwirkung von Bleomycin ist bekannt, daß die Behandlung mit Bleomycin eine Abnahme der Blutplättchen hervorrufen kann, was eine mehr oder weniger starke Thrombocytopenie zur Folge hat (Poliwoda, H. et al. 1982, Beitr. Onkol. 12, 176-181).

Therapeutische Ansätze zum Vermeiden der genannten Nebenwirkungen von Bleomycin, bei denen Medikamente, wie N-Acetylcystein (Ward, H.E. et al. 1987, Pathology 19, 358-360), Ambroxol (Luisetti, H. et al. 1986, Internat. J. Clin. Pharmacol. Res. 6, 129-136) und weitere antithrombische Verbindungen (Poliwoda H. et al. 1982, Beitr. Onkol. 12, 176-181)eingesetzt wurden, zeigten bisher keinen Erfolg.

Hirose et al. (1982, Chest 82, 219) beschreiben die Verwendung von Urokinasen zur Verhinderung der durch Bleomycin verursachten Endothelschädigung und Fibrin- und Kollagenablagerungen im Lungeninterstitium verbunden mit verringerter Gewebe-fibrinolytischer Aktivität in der Lunge. Die Autoren lehren allgemein, daß die durch Bleomycin induzierte Lungenfibrose hauptsächlich aufgrund des Fibrin-Durchtritts und -Ablagerung und seiner fibroblastischen Organisation verursacht wird.

WO97/02230 beschreibt Hydrolasen aus Kaninchenlunge zur Verringerung der Pulmotoxizität von Bleomycin in Menschen und bestimmten Tierarten. Ebenso wird ausgeführt, daß die Hydrolasen die Bleomycine zu nicht oder nur wenig toxischen Produkten abbauen.

Die Offenlegungsschrift DE 1951822 offenbart die Kombination einer Antikrebssubstanz, beispielsweise Bleomycin, und einer Nicht-Antikrebssubstanz, ausgewählt aus Dextransulfat und Urokinase, zur Verhinderung der Nebenwirkungen, die durch die Antikrebssubstanz verursacht werden. Ebenfalls beschrieben wird, daß Lungenfibrosis bei Verwendung von Bleomycin allein beobachtet wird.

Die europäische Patentanmeldung EP 309 602 beschreibt die Verwendung katabolischer Enzyme zum Bekämpfen der erworbenen Immunschwäche (AIDS) und deren Vorstadien (LAS, ARC).

Aufgabe der vorliegenden Erfindung ist es, eine weitere Möglichkeit anzugeben, wie die durch eine Behandlung mit Bleomycin hervorgerufene Thrombocytopenie verringert bzw. vermieden werden kann.

Diese Aufgabe wird erfindungsgemäß gelost durch die Verwendung von mindestens einem hydrolytischen Enzym zur Herstellung eines entsprechenden Arzneimittels.

Überraschenderweise wurde gefunden, daß hydrolytische Enzyme wie Streptokinase, Urokinase, Streptodornase, serratia-Peptidase, Papain, Pankreatin, Bromelain, Chymotrypsin und Trypsin die von Bleomycin hervorgerufene Gefahr einer Thrombocytopenie, verringern bzw. vermeiden können.

Die erfindungsgemäß verwendeten Enzyme sowie die weiteren Bestandteile sind im Handel erhältlich, oder sie können auf einfache Weise aus den folgenden Ausgangsmaterialien isoliert werden.

Streptokinase ist ein Protein von 47 kD, das von Streptokokken produziert wird. Dieses Protein ist ein wirkungsvoller Aktivator des menschlichen Plasminogens, einer inaktiven Vorstufe des Plasmins, das fibrinolytisch wirksam ist.

Urokinase ist ein Enzym mit Molekulargewicht 43 kD aus Niere, das einen Aktivator von Plasminogen darstellt.

Streptodornase ist eine Sammelbezeichnung fur die von Streptococcus ssp. extracellulär gebildeten Desoxyribonukleasen, die teilweise auch gegen RNA aktiv sind.

serratia-Peptidase kann aus einem Mikroorganismus der Gattung Serratia gewonnen werden.

Papain ist ein proteolytisches Enzym, das bis zu den einzelnen Aminosäuren abbaut, und das aus dem Milchsaft von unreifen, fleischigen Früchten des Melonenbaums Carica papaya gewonnen wird. Dieses Enzym kann von Biocon, India Pvt. Ltd., Hebbagodi 562 107 bezogen werden.

Pankreatin wird aus Schweine- oder Rinderpankreas gewonnen und ist ferner erhältlich von Nordmark Arzneimittel, 2082 Uetersen.

Bromelain ist ein proteolytisches Enzym aus dem Preßsaft von Ananas und kann ferner bezogen werden von Taiwan McKay, Biochemical Company Ltd., Chushan.

Chymotrypsin ist ein proteolytisches Enzym aus Pankreas und kann bezogen werden von Biobras Bioquim., do Brasil S.A., Belo Horizonte/MG.

Trypsin ist ebenfalls ein proteolytisches Enzym aus Pankreas und kann bezogen werden von Novo Industri A/S, DK-2880 Bagsvaerd.

Rutin gehört zu den Flavonoidglykosiden und kann bezogen werden von Merck do Brasil.

Thymusfaktoren, die weiterhin den Enzymmischungen zugesetzt werden können, werden aus Thymusdrüsen isoliert.

Die oben genannten hydrolytischen Enzyme sind besonders wirksam, wenn sie in Kombination eingesetzt werden.

Den Kombinationen an Enzymen können vorteilhafterweise zusätzlich Rutin und/oder Thymusfaktoren zugesetzt werden.

Besondere Wirksamkeit bei der erfindungsgemäßen Verwendung zeigt eine Mischung aus den Enzymen Chymotrypsin, Papain und Trypsin, denen zusätzlich ein Hydrolysat aus Kalbsthymus zugesetzt worden ist. Eine ganz besonders wirksame Ausführungsform dieser Zusammensetzung enthält 10 bis 100 mg, vorzugsweise 40 mg Chymotrypsin, 50 bis 500 mg, vorzugsweise 100 mg Papain und 10 bis 100 mg, vorzugsweise 40 mg, Trypsin sowie zusätzlich zu den Enzymen 10 bis 100 mg, vorzugsweise 40 mg, Hydrolysat aus Kalbsthymus.

Ebenfalls besondere Wirksamkeit zeigt eine Mischung aus den Enzymen Chymotrypsin, Papain, Trypsin, Bromelain und Pankreatin, denen zusätzlich Rutin zugesetzt worden ist. Eine ganz besonders wirksame Ausführungsform dieser Zusammensetzung enthält 1 bis 10 mg, vorzugsweise 1 mg, Chymotrypsin, 40 bis 100 mg, vorzugsweise 60 mg, Papain, 10 bis 30 mg, vorzugsweise 24 mg, Trypsin, 20 bis 100 mg, vorzugsweise 45 mg, Bromelain, 50 bis 200 mg, vorzugsweise 100 mg, Pankreatin, sowie als weiteren Zusatz 10 bis 100 mg, vorzugsweise 50 mg, Rutin.

Weiterhin bevorzugt verwendbar ist eine Mischung aus den Enzymen Bromelain und Trypsin, denen zusätzlich Rutin zugesetzt worden ist. Eine besonders wirksame Ausführungsform dieser Zusammensetzung enthält 20 bis 120 mg, vorzugsweise 90 mg, Bromelain, 20 bis 60 mg, vorzugsweise 48 mg, Trypsin, sowie zusätzlich 50 bis 150 mg, vorzugsweise 100 mg, Rutin.

Zur Konfektionierung der Enzyme bzw. Enzymgemische sowie gegebenenfalls weiterer Inhaltsstoffe können übliche Hilfsstoffe zugesetzt werden. Die Konfektionierung kann in an sich bekannter Weise erfolgen.

Die erfindungsgemäß verwendeten hydrolytischen Enzyme bzw. Enzymgemische sind besonders geeignet, um die Gefahr einer von Bleomycin hervorgerufenen Thrombocytopenie zu verringern bzw. zu vermeiden.

Das Beispiel erläutert die Erfindung.

### Beispiel

Verwendung hydrolytischer Enzyme zur Vermeidung bzw. Verringerung der Gefahr einer Thrombocytopenie durch Bleomycin.

Untersucht wurde eine Gruppe von 86 Probanden mit Plattenepithelkarzinom im HNO-Bereich, von denen 54 begleitend zu Bleomycin mit hydrolytischen Enzymen behandelt wurden (3 x 3 Tabletten bis 3 x 5 Tabletten täglich Wobe-Mugos®, Mucos Pharma GmbH & Co.). 32 Patienten wurden mit Bleomycin alleine behandelt. Als Bleomycindosis wurden 105 mg pro Einzeldosis verwendet.

Die Bleomycin-Therapie ohne den zusätzlichen Einsatz hydrolytischer Enzyme führte dazu, daß bei Therapieende die Thrombozytenzahl bei 32 untersuchten Probanden im Durchschnitt nur noch 60 % der ursprünglichen Thrombozytenanzahl vor Therapiebeginn betrug. Wurden jedoch zusätzlich zu Bleomycin hydrolytische Enzyme verwendet, so fiel die Thrombozytenzahl bei 54 untersuchten Probanden bis zu Therapieende im Durchschnitt lediglich auf einen Wert von 90 % der ursprünglichen Anzahl ab.

Wie aus dem obigen Beispiele hervorgeht, kann durch die Verwendung von hydrolytischen Enzymen begleitend zu der Therapie mit Bleomycin eine deutliche Verringerung bzw. eine Vermeidung der Nebenwirkungen von Bleomycin erzielt werden.

## Patentansprüche

1. Verwendung von mindestens einem hydrolytischen Enzym zur Herstellung eines Arzneimittels zur Verringerung bzw. Vermeidung von Nebenwirkungen von Bleomycin, **dadurch gekennzeichnet**, daß die Nebenwirkung von Bleomycin die Gefahr einer Thrombocytopenie ist.

2. Verwendung nach Anspruch 1, **dadurch gekennzeichnet**, daß das bzw. die Enzyme ausgewählt werden aus der Gruppe, umfassend Streptokinase, Urokinase, Streptodornase, Serratia-Peptidase, Papain, Pankreatin, Bromelain, Chymotrypsin und Trypsin.

3. Verwendung nach einem der Ansprüche 1 oder 2, **dadurch gekennzeichnet**, daß zusätzlich Rutin und/oder Thymusfaktoren verwendet werden.

4. Verwendung nach einem der Ansprüche 1 bis 3, **dadurch gekennzeichnet**, daß ein Gemisch aus Chymotrypsin, Papain und Trypsin verwendet wird.

5. Verwendung nach Anspruch 4, **dadurch gekennzeichnet**, daß zusätzlich noch ein Hydrolysat aus Kalbsthymus verwendet wird.

6. Verwendung nach einem der Ansprüche 1 bis 3, **dadurch gekennzeichnet**, daß ein Gemisch aus Chymotrypsin, Papain, Trypsin, Bromelain, Pankreatin und Rutin verwendet wird.

7. Verwendung nach einem der Ansprüche 1 bis 3, **dadurch gekennzeichnet**, daß ein Gemisch aus Bromelain, Trypsin und Rutin verwendet wird.

8. Verwendung nach einem der Ansprüche 1 bis 7, **dadurch gekennzeichnet**, daß zur Konfektionierung der Enzyme und gegebenenfalls weiterer Wirkstoffe übliche Hilfsstoffe zugesetzt werden.

## Claims

1. Use of at least one hydrolytic enzyme in the production of a medicament for reducing or avoiding side-effects of bleomycin, **characterised in that** the side-effect of bleomycin is the risk of a thrombocytopenia.

2. Use according to claim 1, **characterised in that** the enzyme(s) is(are) selected from the group consisting of streptokinase, urokinase, streptodornase, Serratia peptidase, papain, pancreatin, bromelain, chymotrypsin and trypsin.

3. Use according to any one of claims 1 or 2, **characterised in that** rutin and/or thymus factors are additionally used.

4. Use according to any one of claims 1 to 3, **characterised in that** a mixture of chymotrypsin, papain and trypsin is used.

5. Use according to claim 4, **characterised in that** a hydrolysate from calf thymus is additionally used.

6. Use according to any one of claims 1 to 3, **characterised in that** a mixture of chymotrypsin, papain, trypsin, bromelain, pancreatin and rutin is used.

7. Use according to any one of claims 1 to 3, **characterised in that** a mixture of bromelain, trypsin and rutin is used.

8. Use according to any one of claims 1 to 7, **characterised in that** for the confectioning of the enzymes and, optionally, other active ingredients, conventional auxiliary substances are added.

## Revendications

1. Utilisation d'au moins une enzyme hydrolytique pour préparer un médicament destiné à réduire ou éviter les effets secondaires de la bléomycine, caractérisée en ce que l'effet secondaire de la bléomycine est un risque de thrombocytopénie.

2. Utilisation selon la revendication 1, caractérisée en ce que la ou les enzymes sont choisies dans l'ensemble comprenant la streptokinase, l'urokinase, la streptodornase, la serratia-peptidase, la papaïne, la pancréatine, la bromélaïne, la chymotrypsine et la trypsine.

3. Utilisation selon l'une des revendications 1 ou 2, caractérisée en ce qu'on utilise en outre de la rutine et/ou des facteurs du thymus.

4. Utilisation selon l'une des revendications 1 à 3, caractérisée en ce qu'on utilise un mélange de chymotrypsine, de papaïne et de trypsine.

5. Utilisation selon la revendication 4, caractérisée en ce qu'on utilise en outre encore un hydrolysat de thymus de veau.

6. Utilisation selon l'une des revendications 1 à 3, caractérisée en ce qu'on utilise un mélange de chymotrypsine, de papaïne, de trypsine, de bromélaïne, de pancréatine et de rutine.

7. Utilisation selon l'une des revendications 1 à 3, caractérisée en ce qu'on utilise un mélange de bromélaïne, de trypsine es de rutine.

8. Utilisation selon l'une des revendications 1 à 7, caractérisée en ce que, pour confectionner les enzymes et éventuellement les autres principes actifs, on ajoute des adjuvants usuels.
